# EUROPÄISCHE PATENTANMELDUNG

(11) **EP 1 462 075 A1**
(43) Veröffentlichungstag der Anmeldung: **29.09.2004**
(21) Anmeldenummer: 03006534.6
(22) Anmeldetag: 24.03.2003
(51) Int. Cl.: A61F 13/00, A61F 15/00

(54) **Mittel zur Fixierung von Wundauflagen auf der Haut**

(71) Anmelder: IVF Hartmann AG, 8212 Neuhausen am Rheinfall (CH)
(72) Erfinder: Ha, Suk-Woo, 8246 Langwiesen (CH)
(74) Vertreter: Blum, Rudolf Emil Ernst

(57) **Zusammenfassung**

Es wird ein Wundverband beschrieben, der mindestens einen Befestigungsteil (2) und einen Abdeckteil (3) umfasst, wobei der mindestens eine Befestigungsteil (2) auf der einen Seite mindestens stellenweise eine auf der Haut (6) klebende Schicht (22) umfassend einen hautfreundlichen Klebstoff und auf der anderen Seite mindestens stellenweise den einen Teil eines Klettverschlusses (21) aufweist und der Abdeckteil (3) in seinem Randbereich mindestens stellenweise den anderen Teil eines Klettverschlusses (31) aufweist, derart dass der eine Teil des Klettverschlusses (21) und der andere Teil des Klettverschlusses (31) so positioniert sind, dass sie mindestens stellenweise zur Herstellung einer Klettverbindung (4) befähigt sind.

## Beschreibung

Die vorliegende Erfindung betrifft ein Mittel zur Fixierung von Wundauflagen auf der Haut, insbesondere ein Mittel, das auch für die Fixierung von feuchten und nassen Wundauflagen geeignet und hautschonend ist.

Zum einfachen Abdecken kleinerer Wunden werden heute Wundverbände eingesetzt, die eine unter Anwendung von Fingerdruck klebende Schicht aufweisen. Das Problem mit diesen Wundverbänden ist, dass die Klebefähigkeit im feuchten Zustand - falls überhaupt noch vorhanden - mangelhaft ist. Insbesondere bei den heute immer mehr aufkommenden feuchten und nassen Wundauflagen ist dies sehr ärgerlich, da bei deren Applikation spezielle Vorsichtsmassnahmen ergriffen werden müssen.

Ferner können trotz hautschonender Klebstoffe bei mehrmaligem Entfernen und wieder Aufbringen des Wundverbandes im Bereich der Klebung unerwünschte Hautirritationen auftreten.

Es war deshalb ein Ziel dieser Erfindung, einen Wundverband bereitzustellen, der für das Aufbringen feuchter und nasser Wundauflagen geeignet ist und/oder der bei mehrmaligem Wechsel der Wundauflage die Haut weniger beansprucht.

Dieses Ziel wurde erreicht durch Bereitstellen eines Wundverbandes gemäss Anspruch 1. Weitere Aspekte dieser Erfindung sind in den anderen unabhängigen Ansprüchen geschützt und spezielle Ausführungsarten sind den abhängigen Ansprüchen zu entnehmen.

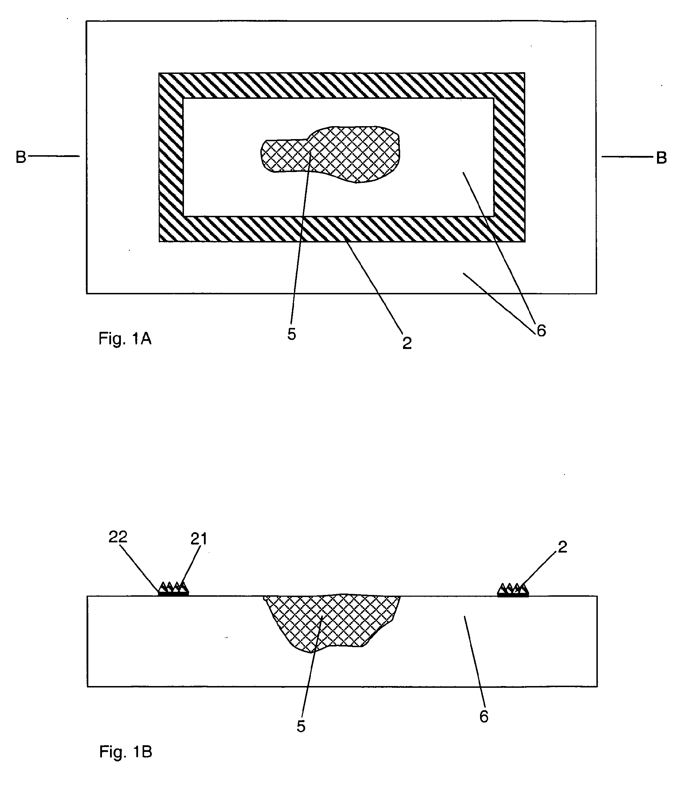

Figur 1A zeigt den ersten Schritt bei der Anwendung des erfindungsgemässe Wundverbandes in Aufsicht.

Figur 1B ist ein Querschnitt durch den in Figur 1A gezeigten Zustand entlang der Linie B-B.

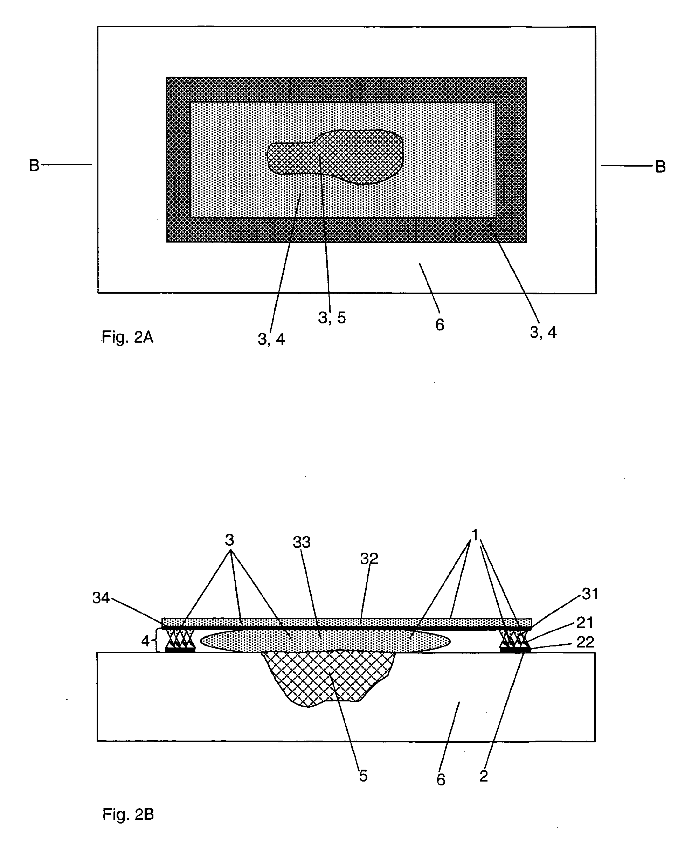

Figur 2A ist eine Aufsicht auf einen durchsichtig gestalteten, vollständigen erfindungsgemässen Wundverband nach Ausführung des zweiten Schrittes.

Figur 2B ist ein Querschnitt durch den Wundverband gemäss Figur 2A entlang der Linie B-B.

Der erfindungsgemässe Wundverband 1 zeichnet sich dadurch aus, dass er mindestens einen Befestigungsteil 2, üblicherweise ein selbsthaftendes Teil, das als "selbstklebendes Klettpflaster" bezeichnet werden kann, und einen Abdeckteil 3 umfasst, wobei der mindestens eine Befestigungsteil 2 auf der einen Seite mindestens stellenweise eine auf der Haut klebende Schicht 22 umfassend einen hautfreundlichen Klebestoff und auf der anderen Seite mindestens stellenweise den einen Teil eines Klettverschlusses 21 aufweist, und der Abdeckteil 3 in seinem Randbereich mindestens stellenweise den anderen Teil eines Klettverschlusses 31 aufweist, derart dass der eine Teil des Klettverschlusses 21 und der andere Teil des Klettverschlusses 31 so positioniert sind, dass sie mindestens stellenweise zur Herstelung einer Klettverbindung 4 befähigt sind.

Üblicherweise umfasst der erfindungsgemässe Wundverband 1 ein Abdeckteil 3 mit einer Deckschicht 32 und einer Wundauflage 33, insbesondere einer feuchten oder gar nassen Wundauflage 33.

Je nach Anwendung können im Wundverband 1 die Wundauflage 33 und die Deckschicht 32 getrennt voneinander vorliegen oder fest miteinander verbunden sein, wobei für das einfache Aufbringen des Wundverbands 1 auf die Haut 6 die verbundene Ausführungsform bevorzugt ist.

Die Wundauflage 33 kann in trockener, feuchter oder nasser Form auf die Wunde 5 gelegt und mit dem Befestigungsteil 2, insbesondere dem Klettpflaster, befestigt werden. Es wird kein zusätzlicher Verband benötigt.

Die Deckschicht kann okklusiv, semi-okklusiv oder wasserdampfdurchlässig sein. Bei Bedarf kann eine atmungsaktive Deckschicht gewählt werden.

Die Wundauflage 33 und/oder die Deckschicht 32 können transparent oder nicht transparent sein. Beispielsweise kann bei Vorliegen einer transparenten Wundauflage 33 zur Gewährleistung der optischen Kontrolle der Wundheilung eine transparente Deckschicht gewählt werden.

Geeignete Materialien für die Deckschicht 32 sind Vliese oder Polymerfolien wie Polyurethanfolien.

Als Wundauflage 33 geeignet sind z.B. eine Vielzahl kommerziell erhältlicher Wundauflagen.

In einer Ausführungsform weist die Deckschicht 32 einseitig zumindest im Bereich der Wundauflage 33 und des Teils des Klettverschlusses 31 eine Klebstoffschicht 34 auf, wobei diese Klebstoffschicht 34 einen oder mehrere Klebstoffe enthalten kann, z. B. einen Klebstoff für die Befestigung der Wundauflage 33 und einen anderen Klebstoff für die Befestigung des einen Teils des Klettverschlusses 31. Geeignete Klebstoffe sind nichttoxisch und dem Fachmann bekannt. Sie umfassen u.a. Klebstoffe auf der Basis von Acrylaten, Polyurethanen, Silikonen oder Hydrokolloiden.

Der eine Teil des Klettverschlusses 31 und/oder die Wundauflage 33 können zu deren Fixierung statt mit der Deckschicht 32 verklebt mit dieser verschweisst oder vernäht werden.

Üblicherweise ist der Befestigungsteil 2, das selbstklebende Klettpflaster, auf der einen Seite mit einer hautfreundlichen Klebstoffschicht 22 versehen, insbesondere einer Klebstoffschicht, die unter Fingerdruck auf der Haut 6 befestigt werden kann. Geeignete Klebstoffe, die sowohl an kommerziell erhältlichen Klettverschlüssen 21 haften als auch ablösbar auf der Haut, sind z.B. Klebstoffe auf der Basis von Acrylaten, Polyurethanen, Silikonen oder Hydrokolloiden.

Eine solche Klebstoffschicht wird zum Schutz vor unerwünschter Klebung resp. Verschmutz im Zeitraum zwischen Produktion und Anwendung üblicherweise mit einer ablösbaren Schutzschicht versehen, z.B. einem silikonisierten Papier.

Geeignete Klettverschlüsse 21, 31 sind solche, die möglichst fein sind, damit der Wundverband nicht zu gross resp. dick wird. Solche Klettverschlüsse 21,31 sind bekannt unter der Bezeichnung MICROPLAST® und z.B. erhältlich von Berlinger & Co. AG. Solche Klettverbindungen 21, 31 können aus unterschiedlichen Materialkombinationen bestehen, z.B. der eine Teil des Klettverschlusses (z.B. 31) aus Polypropylen (PP) und der andere Teil (z.B. 21) aus Polyamid.

Für viele Anwendungen ist es nicht notwendig, dass die Klettverbindung 4 den ganzen Rand des Wundverbandes 1 befestigt, obschon eine über den ganzen Rand des Wundverbandes 1 gehende Befestigung bevorzugt ist. Meist sind 4 bis 8 Stellen ausreichend, z.B. bei einem vierekkigen Verband alle Ecken, sowie gegebenenfalls die Seitenmitten. Bei einer solchen Ausführungsform kann es vorteilhaft sein, die mit Klettverschluss versehenen Bereiche 21 des Befestigungsteils 2 grösser zu dimensionieren als die entsprechenden Stellen des Abdeckteils 3. Dadurch wird die Positionierung erleichtert und durch die nur stellenweise Herstellung einer Klettverbindung erhält der Wundverband 1 eine gewisse Anpassungsfähigkeit an Bewegungen.

Je nach Anwendung ist es vorteilhaft, wenn der erfindungsgemässe Wundverband 1 nicht in Form eines Befestigungsteils 2 zusammen mit einem Abdeckteil 3 in den Handel kommt, sondern in Form eines Kits. Ein solcher Kit kann beispielsweise mehrere Abdeckteile 3 umfassen und den Befestigungsteil im "Endlos"-Format, z.B. ein längeres Stück oder eine Rolle, von der Befestigungsteile abgeschnitten werden können. Dies ist z.B. bei einer nur auf gewisse Stellen beschränkten Klettverbindung 4 günstig.

Ein solcher Kit umfasst deshalb
a) mindestens einen Befestigungsteil 2, der auf der einen Seite eine auf der Haut klebende Schicht 22 umfassend einen hautfreundlichen Klebstoff und auf der anderen Seite mindestens stellenweise den einen Teil eines Klettverschlusses 21 aufweist, und
b) mindestens einen Abdeckteil 3, der in seinem Randbereich mindestens stellenweise den anderen Teil eines Klettverschlusses 31 aufweist.

Anstelle eines Kits können auch der Befestigungsteil 2 und der Abdeckteil 3 getrennt voneinander vorliegen resp. verkauft werden.

Ein erfindungsgemässer Wundverband 1 lässt sich einfach mittels dem Fachmann bekannten Verfahren herstellen.

Der erfindungsgemässe Wundverband 1 wird beispielsweise angewendet, indem ein dem Randbereich des Abdeckteils 3 entsprechender Befestigungsteil 2, insbesondere ein selbsthaftendes Klettpflaster, um die Wunde gelegt und direkt auf die Haut geklebt wird. Alternativ können auch mehrere kleinere Befestigungsteile 2 so auf die Haut geklebt werden, dass sie die Befestigung des Abdeckteils 3 an ausreichend vielen Stellen gestatten, um eine gute Haftung zu gewährleisten.

Das Aufkleben des Befestigungsteils 2 auf die Haut erfolgt vorzugsweise nach der Wundreinigung und Desinfektion. Der Befestigungsteil 2, das Klettpflaster, kann jedoch auch vorher angebracht werden.

Durch die Verwendung eines Befestigungsteils 2, eines Klettpflasters, kann eine trockene, feuchte oder nasse Wundauflage 33 resp. ein eine solche Wundauflage 33 umfassender Abdeckteil 3 mehrfach angebracht resp. erneuert werden, ohne dass die Klebung auf der Haut erneuert werden muss, was zur Schonung der Haut beiträgt. Ferner lässt sich durch die Trennung von Befestigungsteil 2 und Abdeckteil 3 im trockenen Zustand eine Klebeverbindung zwischen Haut und Befestigungsteil 2 herstellen, die durch das nachträgliche Anbringen einer feuchten oder gar nassen Wundauflage 33 nicht (mehr) beeinträchtigt wird.

Nachdem der Befestigungsteil 2 auf der Haut angeklebt worden ist, wird erst die Wundauflage 33 auf die Wunde aufgebracht und anschliessend mit der im Randbereich den zum Befestigungsteil 2 korrespondierenden Teil des Klettverschlusses 31 aufweisenden Deckschicht 32 abgedeckt. Der Randbereich der Deckschicht 32 wird dann an den Befestigungsteil 2 resp. die Befestigungsteile 2 angedrückt, so dass eine feste Klettverbindung 4 entsteht.

In einer bevorzugten Ausführungsform ist die Wundauflage 33 fest mit der Deckschicht 32 verbunden, insbesondere mittels einer Klebstoffschicht 34 verklebt oder verschweisst oder vernäht.

Falls keine visuelle Kontrolle möglich ist, wird die Wundauflage 33 resp. der Abdeckteil 3 nach einer vorbestimmten Zeit entfernt, um den Fortschritt der Wundheilung zu kontrollieren. Eine weitere Reinigung und/oder Desinfektion kann erfolgen. Der Befestigungsteil 2, insbesondere das Klettpflaster, muss dabei nicht entfernt werden. Bei Bedarf kann die bisherige Wundauflage 33 resp. der Abdeckteil 3 auf dem Klettpflaster wieder angebracht oder eine weitere, neue Wundauflage 33 resp. ein neuer Abdeckteil 3 darauf appliziert werden.

Nach erfolgter Wundheilung werden die Wundauflage 33 resp. der Abdeckteil 3 und der Befestigungsteil 2, insbesondere das Klettpflaster, gemeinsam oder vorzugsweise erst der Abdeckteil 3 und anschliessend der Befestigungsteil 2 entfernt.

## Patentansprüche

1. Wundverband (1), **dadurch gekennzeichnet, dass** er mindestens einen Befestigungsteil (2) und einen Abdeckteil (3) umfasst, wobei der mindestens eine Befestigungsteil (2) auf der einen Seite mindestens stellenweise eine auf der Haut klebende Schicht umfassend einen hautfreundlichen Klebstoff und auf der anderen Seite mindestens stellenweise den einen Teil eines Klettverschlusses (21) aufweist und der Abdeckteil (3) in seinem Randbereich mindestens stellenweise den anderen Teil eines Klettverschlusses (31) aufweist, derart dass der eine Teil des Klettverschlusses (21) und der andere Teil des Klettverschlusses (31) so positioniert sind, dass sie mindestens stellenweise zur Herstellung einer Klettverbindung (4) befähigt sind.

2. Wundverband (1) gemäss Anspruch 1, **dadurch gekennzeichnet, dass** der Abdeckteil (3) eine Deckschicht (32) und eine Wundauflage (33) umfasst.

3. Wundverband (1) gemäss einem der vorangehenden Ansprüche, **dadurch gekennzeichnet, dass** die Wundauflage (33) und die Deckschicht (32) fest miteinander verbunden sind.

4. Wundverband (1) gemäss einem der vorangehenden Ansprüche, **dadurch gekennzeichnet, dass** die Deckschicht (32) einseitig zumindest im Bereich der Wundauflage (33) und/oder des Teils des Klettverschlusses (31) eine Klebstoffschicht (34) aufweist.

5. Wundverband (1) gemäss einem der vorangehenden Ansprüche **dadurch gekennzeichnet, dass** der Befestigungsteil (2) auf der Seite der Klebstoffschicht (22) mit einer ablösbaren Schutzschicht versehen ist.

6. Wundverband (1) gemäss einem der vorangehenden Ansprüche **dadurch gekennzeichnet, dass** der eine Teil des Klettverschlusses (21) und der andere Teil des Klettverschlusses (31) so positioniert sind, dass sie zur Herstellung einer Klettverbindung (4) in mindestens zwei, vorzugsweise mindestens vier Bereichen befähigt sind.

7. Wundverband (1) gemäss einem der vorangehenden Ansprüche **dadurch gekennzeichnet, dass** die auf der Haut klebende Schicht (22) die ganze, bei Applikation des Befestigungsteils (2) mit der Haut (6) in Kontakt stehende Seite des Befestigungsteils (2) bedeckt.

8. Wundverband (1) gemäss einem der vorangehenden Ansprüche **dadurch gekennzeichnet, dass** der Befestigungsteil (2) dem Umfang resp. dem ganzen, zumindest bereichsweise mit dem einen Teil des Klettverschlusses (31) versehenen Randbereich des Abdeckteils (3) entspricht.

9. Verwendung eines Klettverschlusses zur Herstellung eines Wundverbandes (1) gemäss einem der vorangehenden Ansprüche.

10. Kit, **dadurch gekennzeichnet, dass** er
a) mindestens einen Befestigungsteil (2), der auf der einen Seite mindestens stellenweise eine auf der Haut klebende Schicht (22) umfassend einen hautfreundlichen Klebestoff und auf der anderen Seite mindestens stellenweise den einen Teil eines Klettverschlusses (21) aufweist, und
b) mindestens einen Abdeckteil (3), der in seinem Randbereich mindestens stellenweise den anderen Teil eines Klettverschlusses (31) aufweist,
umfasst, wobei der Abdeckteil (3) mit mindestens einem Befestigungsteil (2) einen Wundverband gemäss einem der Ansprüche 1 bis 8 bilden können.

11. Befestigungsteil (2), wie in einem der Ansprüche 1 bis 8 definiert.

12. Abdeckteil (3), wie in einem der Ansprüche 1 bis 8 definiert.
